(19) Europäisches Patentamt
European Patent Office
Office européen des brevets

(11) EP 1 669 057 A1

(12) # DEMANDE DE BREVET EUROPEEN

(43) Date de publication:
**14.06.2006 Bulletin 2006/24**

(51) Int Cl.:
***A61K 8/18*** *(2006.01)*

(21) Numéro de dépôt: **05292051.9**

(22) Date de dépôt: **03.10.2005**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **16.11.2004 FR 0452636**

(71) Demandeur: **L'OREAL**
**75008 Paris (FR)**

(72) Inventeurs:
- **Ricard, Audrey**
**75012 Paris (FR)**
- **Le Merrer, Carole**
**75011 Paris (FR)**

(74) Mandataire: **Chantraine, Sylvie Hélène**
**L'OREAL - D.I.P.I.**
**25-29, Quai Aulagnier**
**92600 Asnieres (FR)**

(54) **Composition cosmétique contenant un éther de polyol et un ester**

(57) La présente demande concerne une composition comprenant une phase grasse comportant au moins un éther de polyol et au moins un ester d'acide néo.

L'éther de polyol peut être notamment un éther de pentaérythritol.

Cette composition peut constituer notamment un stick et elle peut être utilisée en tant que produit de soin et/ou de maquillage des matières kératiniques, notamment de la peau, des lèvres et/ou des phanères.

EP 1 669 057 A1

**Description**

La présente invention a trait à une composition comprenant une phase grasse comportant au moins un éther de polyol particulier. La composition peut contenir au moins un ester hydrocarboné, par exemple un ester d'acide néo. Cette composition est susceptible d'être utilisée comme composition de maquillage et/ou de soin des matières kératiniques telles la peau, les lèvres et les phanères.

La demande EP 1 306 074 décrit une composition solide contenant un éther de polyol, une huile et une cire de polarité inverse. Ce document décrit comment cette association permet d'obtenir une composition solide qui ne fond pas à une température inférieure ou égale à 37°C, tout en s'étalant aisément sur la peau pour fournir un bon dépôt.

L'objet de la présente invention est de fournir une composition cosmétique qui peut être sous la forme d'un stick ou d'une pâte. Cette composition présente l'avantage d'avoir une meilleure tenue sur les matières kératiniques que les compositions de l'art antérieur contenant un éther de polyol, tout en ayant un bon niveau de confort.

La présente invention a pour objet une composition cosmétique comprenant dans un milieu physiologiquement acceptable, au moins une phase grasse comportant au moins un éther de polyol choisi parmi les éthers de pentaérythritol et de polyalkylène glycol, et au moins un ester hydrocarboné.

La composition de l'invention comprend de préférence une seule phase grasse, et cette phase grasse est de préférence une phase continue.

Les compositions de l'invention peuvent être des compositions anhydres, et comprendre alors jusqu'à 10 % en poids de phase hydrophile par rapport au poids total de la composition, de préférence 1 à 5 % en poids de phase hydrophile et mieux de 1 à 2 % de phase hydrophile par rapport au poids total de la composition, la phase hydrophile étant constituée d'eau seule, ou d'eau et d'additifs hydrophiles et hydrosolubles tels que polyols, gélifiants et/ou actifs. Si cette phase hydrophile est présente, elle est de préférence dispersée dans la phase grasse qui forme une phase continue.

Selon un mode particulier de réalisation de l'invention, la composition est anhydre, c'est-à-dire qu'elle ne comporte que la phase grasse, ou quasiment anhydre, c'est-à-dire qu'elle comporte moins de 5 % en poids d'eau et/ou additifs hydrophiles ou hydrosolubles.

L'invention a pour premier objet une composition comprenant dans un milieu physiologiquement acceptable, au moins une phase grasse comportant au moins un éther de polyol, choisi parmi les éthers de pentaérythritol et de polyalkylène glycol, et au moins un ester résultant de la réaction d'un alcool avec un acide carboxylique de formule (I) suivante :

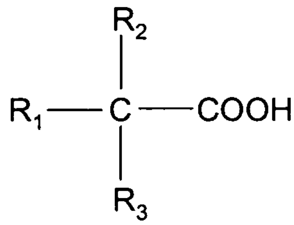

(I)

dans laquelle $R_1$, $R_2$ et $R_3$ sont des radicaux choisis indépendamment parmi les radicaux alkyle, aryle, aralkyle, et leurs associations,

ledit acide carboxylique de formule (I) étant nommé dans la suite du texte « acide néo ».

L'invention a pour autre objet une composition comprenant dans un milieu physiologiquement acceptable, au moins une phase grasse comportant au moins un éther de polyol, au moins un ester hydrocarboné et une huile non volatile différente dudit ester hydrocarboné.

Selon un autre aspect, l'invention a pour objet une composition comprenant dans un milieu physiologiquement acceptable, au moins une phase grasse comportant au moins un éther de polyol et aux moins deux esters hydrocarbonés.

Selon cet aspect, l'un des deux esters peut être un ester d'acide néo de formule (I) décrite précédemment. L'autre des deux esters peut être un mono-ester comprenant notamment de 18 à 26 atomes de carbone. Avantageusement l'ester d'acide néo et le mono-ester sont dans une proportion massique comprise entre 2/1 et 1/5, notamment comprise entre 1/1 et 1/5, de préférence entre 1/1 et 1/3, de préférence encore de l'ordre de 1/2.

Selon cet aspect de l'invention, l'ester d'acide de formule (I) peut être présent dans la composition à une concentration comprise entre 5 et 35%, notamment entre 10 et 15%. L'autre ester hydrocarboné peut être présent à une concentration comprise entre 10 et 50% en poids, notamment entre 15 et 30% en poids par rapport au poids total de la composition.

Selon un autre de ses aspects, l'invention a pour objet une composition comprenant dans un milieu physiologiquement acceptable, au moins une phase grasse comportant au moins un éther de polyol, un composé pâteux autre que ledit éther de polyol, et au moins un ester hydrocarboné.

ETHER DE POLYOL

Dans la composition selon l'invention, l'éther de polyol peut être choisi notamment parmi les éthers de pentaérythritol et de polyalkylène glycol, les éthers d'alcool gras et de sucre, et leurs mélanges.

Les éthers de pentaérythritol et de polyalkylène glycol peuvent comporter notamment de 1 à 450 motifs oxyalkylénés, de préférence de 1 à 200 motifs oxyalkylénés, mieux 1 à 100 motifs oxyalkylénés et encore mieux de 1 à 50 motifs oxyalkylénés. Ils peuvent être en particulier choisis parmi les éthers de pentaérythritol et de polyéthylène glycol comportant de 1 à 450 motifs oxyéthylénés, de préférence de 1 à 200 motifs oxyéthylénés, mieux 1 à 100 motifs oxyéthylénés et encore mieux de 1 à 50 motifs oxyéthylénés ; les éthers de pentaérythritol et de polypropylène glycol comportant de 1 à 450 motifs oxypropylénés, de préférence de 1 à 200 motifs oxypropylénés, mieux 1 à 100 motifs oxypropylénés et encore mieux de 1 à 50 motifs oxypropylénés ; et leurs mélanges. Selon un mode préféré de réalisation de l'invention, on utilise l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société Vevy, mélange où les constituants se trouvent dans un rapport en poids 46/46/8 : 46 % de PEG-5 Pentaerythrityl Ether, 46 % de PPG-5 Pentaerythrityl Ether et 8 % d'huile de soja.

Les éthers d'alcool gras et de sucre peuvent être choisis dans le groupe comprenant les éthers ou mélanges d'éthers d'alcool gras en $C_8$-$C_{22}$ et de glucose, de maltose, de sucrose ou de fructose ; les éthers ou mélanges d'éthers d'alcool gras en $C_{14}$-$C_{22}$ et de méthylglucose ; et leurs mélanges.

Les alcools gras en $C_8$-$C_{22}$ ou en $C_{14}$-$C_{22}$ formant le motif gras des éthers de sucre comportent une chaîne alkyle linéaire saturée ou non saturée, comportant respectivement de 8 à 22 ou de 14 à 22 atomes de carbone. Le motif gras des éthers provenant de l'alcool gras peut être notamment choisi parmi les motifs décyle, cétyle, béhényle, arachidyle, stéaryle, palmityle, myristyle, lauryle, capryle, hexadécanyle, et leurs mélanges tels que cétéaryle (mélange de cétyle et de stéaryle).

A titre d'exemples d'éthers d'alcool gras et de sucre, on peut citer en particulier les alkylpolyglucosides (APG) tels que le décylglucoside et le laurylglucoside commercialisés par exemple par la société Henkel sous les dénominations respectives Plantaren 2000 et Plantaren 1200, le cétéarylglucoside éventuellement en mélange avec l'alcool cétostéarylique, le dit mélange étant commercialisé par exemple sous la dénomination Montanov 68 par la société Seppic (mélange où les constituants se trouvent dans un rapport 12/46/42 : 12 % de Cétéarylglucoside, 46 % d'alcool cétylique et 42 % d'alcool stéarylique). Comme cétéarylglucoside, on peut citer aussi les produits commercialisés sous la dénomination Tegocare CG90 par la société Goldschmidt et sous la dénomination Emulgade KE3302 par la société Henkel. Comme APG, on peut citer aussi l'arachidyl glucoside, par exemple sous la forme du mélange d'alcools arachidique et béhénique et d'arachidylglucoside, commercialisé sous la dénomination Montanov 202 par la société Seppic. On peut citer aussi les alkylpolyglucosides à chaîne ramifiée ou insaturée, tels que l'isostéarylglucoside éventuellement en mélange avec l'alcool isostéarylique, commercialisé par exemple sous la dénomination Montanov WO18 par la société Seppic, et l'oléylglucoside éventuellement en mélange avec l'alcool oléylique, commercialisé par la société Seppic. On peut utiliser aussi un mélange de ces alkylpolyglucosides. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

La quantité d'éther(s) de polyol peut varier dans une large mesure, et elle peut aller par exemple de 0,5 à 40 % en poids, de préférence de 1 à 30 % en poids et mieux de 5 à 25 % en poids par rapport au poids total de la composition.

## ESTER HYDROCARBONE

Par « ester hydrocarboné», on entend un composé comprenant au moins une fonction ester COO.
Le mot « ester », selon l'invention, signifie un monoester, un diester, un triester ou plus généralement un polyester. L'ester de la composition selon l'invention peut résulter d'une estérification totale ou partielle (dans ce dernier cas, l'ester comporte une ou plusieurs fonctions -OH libre).

Par « au moins » un ester, on entend un ou plusieurs esters.

Par « physiologiquement acceptable », on entend non toxique et susceptible d'être appliquée sur la peau, les phanères ou les lèvres d'êtres humains.

Le milieu physiologiquement acceptable de la composition selon l'invention est en particulier cosmétiquement acceptable, c'est à dire agréable de goût, de toucher, d'aspect et/ou d'odeur, applicable plusieurs jours pendant plusieurs mois.

L'ester hydrocarboné conforme à l'invention est de préférence une huile, c'est-à-dire un corps gras liquide à pression atmosphérique et à une température de 23°C.

L'ester hydrocarboné peut être linéaire, ramifié ou cyclique, saturé ou insaturé.

L'ester hydrocarboné est de préférence une huile non volatile. Par "huile non volatile", on entend une huile (ou milieu non aqueux) ayant une pression de vapeur inférieure à 0,13 Pa.

En particulier, l'ester hydrocarboné peut répondre à la formule RCOOR' dans laquelle RCOO représente un reste d'acide carboxylique comportant de 2 à 30 atomes de carbone, et R' représente une chaîne hydrocarbonée contenant de 1 à 30 atomes de carbone.

RCOO est de préférence le reste d'un acide carboxylique aliphatique comprenant 2 à 30 atomes de carbone, de préférence de 4 à 26 atomes de carbone, de préférence encore de 4 à 22 atomes de carbone.

Le radical R est avantageusement un radical alkyle ou un radical alkényle. On entend par radical alkyle, un radical aliphatique constitué de carbone et d'hydrogène, linéaire ou ramifié, et saturé. On entend par radical alkényle un radical aliphatique constitué de carbone et d'hydrogène, linéaire ou ramifié, et insaturé, i.e. comportant au moins une double liaison carbone carbone, de préférence de une à trois doubles liaisons, de préférence une double liaison carbone carbone.

RCOO peut représenter le reste d'un acide gras, c'est-à-dire un acide obtenu par hydrolyse de corps gras d'origine végétale ou animale.

Le radical R'O représente le reste d'un alcool, de préférence un alcool aliphatique, saturé ou insaturé, linéaire ou ramifié. Le radical R' est avantageusement un radical alkyle ou un radical alkényle, indépendamment du choix du radical R, alkyle et alkényle étant définis comme précédemment.

R'O peut représenter le reste d'un alcool gras, c'est-à-dire d'un alcool obtenu par hydrogénation d'un acide gras tel que défini précédemment.

R'O est de préférence le reste d'un alcool aliphatique comprenant 2 à 30 atomes de carbone, de préférence de 4 à 26 atomes de carbone, de préférence encore de 4 à 22 atomes de carbone.

R et R' sont choisis indépendamment l'un de l'autre. De préférence, on les choisit de manière à ce qu'ils soient tous les deux saturés et ramifiés, ou tous les deux linéaires et mono insaturés.

L'ester peut notamment comprendre jusqu'à 60 atomes de carbone, de préférence de 10 à 45, et de préférence de 18 à 40 atomes de carbone.

Selon un mode de mise en oeuvre, l'ester hydrocarboné est un mono-ester ramifié et saturé. De préférence l'ester est un mono-ester d'un acide carboxylique aliphatique ramifié et saturé et d'un alcool aliphatique ramifié et saturé.

Selon un autre mode de mise en oeuvre, l'ester hydrocarboné est un mono-ester d'un acide gras mono-insaturé (comportant une double liaison carbone carbone) et linéaire, et d'un alcool gras mono-insaturé et linéaire.

Ainsi, les esters peuvent être choisis parmi une liste non limitative comprenant

- les esters de l'acide isononanoïque comme l'isononanoate d'isononyle, l'isononanoate d'octyle, l'isononanoate d'isodécyle, l'isononanoate d'isotridécyle, l'isononanoate d'isostéaryle,
- les esters de l'alcool isopropylique, tels que le myristate d'isopropyle, le palmitate d'isopropyle, le stéarate ou l'isostéarate d'isopropyle,
- les esters d'acides gras comme l'huile de purcellin, le palmitate d'éthyle, le stéarate d'octyle ;
- les esters hydroxylés comme le lactate d'isostéaryle, l'hydroxystéarate d'octyle, l'adipate de diisopropyle,
- les heptanoates, octanoates, décanoates d'alcool gras, comme l'octanoate de cétyle et l'octanoate de tridécyle
- et leurs mélanges.

L'ester hydrocarboné peut également être choisi parmi les esters d'acide néo. Par « ester d'acide néo», on entend un ester résultant de la réaction d'un alcool avec un acide carboxylique de formule (I) suivante :

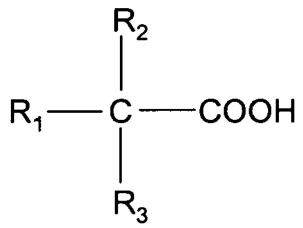

(I)

dans laquelle $R_1$, $R_2$ et $R_3$ sont des radicaux choisis indépendamment parmi les radicaux alkyle, aryle, aralkyle, éventuellement fonctionnalisés, et leurs associations,

Les esters d'acide carboxylique néo, peuvent être choisis parmi les esters de l'acide néopentanoïque comme le néopentanoate d'isodécyle, le néopentanoate d'isotridécyle, le néopentanoate d'isostéaryle et le néopentanoate d'octyl-2-docécyle.

Le carbone situé en alpha de la fonction acide de l'acide carboxylique de formule (I) est trisubstitué par des radicaux choisis indépendamment parmi les radicaux alkyle, aryle, aralkyle, et leurs associations, ces radicaux pouvant être identiques ou différents. Cet atome de carbone est donc directement lié à 4 atomes de carbone et est appelé carbone « néo ». Un acide comportant une telle structure est appelé acide « néo » et sera désigné ainsi dans la suite du texte.

Par radical alkyle, on entend une chaîne hydrocarbonée aliphatique saturée ou insaturée, ramifiée ou non ramifiée, comportant notamment de 1 à 28 atomes de carbone.

Le mot « ramifié » signifie au moins une chaîne pendante hydrocarbonée comportant au moins un atome de carbone.

Par radical aryle, on entend un radical dérivé d'un composé cyclique aromatique par élimination d'un atome d'hydrogène tel que par exemple les radicaux phényle ou tolyle.

Par radical aralkyle, on entend une chaîne alkyle substituée par un radical aryle, par exemple du type R'-$C_6H_5$, R' étant un alkyle en $C_1$-$C_5$, comme par exemple les radicaux benzyl ou phénéthyle.

Ces radicaux alkyle, aryle ou aralkyle peuvent être fonctionnalisés ce qui signifie qu'ils peuvent comprendre dans leur structure un hétéroatome, c'est-à-dire un atome différent de l'atome de carbone (tel qu'un atome d'oxygène, d'azote, de soufre, de fluor) ou un groupement fonctionnel tel qu'un groupement COOH, OH, NHR ou COOR.

L'acide carboxylique « néo » peut être un monoacide ou un polyacide, à savoir que au moins un des radicaux $R_1$, $R_2$ ou $R_3$ contient un groupement fonctionnel -COOH. De préférence, l'acide carboxylique est un monoacide.

De façon préférentielle, les radicaux $R_1$, $R_2$ et $R_3$ sont choisis indépendamment parmi les radicaux alkyle, aryle ou aralkyle non fonctionnalisés.

Avantageusement, les radicaux $R_1$, $R_2$ et $R_3$ sont choisis indépendamment parmi les radicaux alkyle saturés, de préférence parmi les radicaux alkyle saturés en $C_1$- $C_{15}$, et mieux, en $C_1$- $C_6$ comme par exemple le radical méthyle, éthyle, propyle, isopropyle, pentyle, hexyle.

Avantageusement, l'acide carboxylique « néo » comprend un nombre total d'atomes de carbone allant de 5 à 30, et mieux de 5 à 15.

On utilise préférentiellement un acide carboxylique « néo » choisi parmi l'acide néopentanoïque de formule $CH_3$-$C(CH_3)_2$-COOH, l'acide néoheptanoïque de formule $C_3H_7$-$C(CH_3)_2$-COOH, l'acide néodécanoïque de formule $C_6H_{13}$-$C(CH_3)_2$-COOH, et leurs mélanges. De manière plus préférentielle, l'acide carboxylique « néo » est l'acide néopentanoïque.

L'ester de la composition selon l'invention résulte de la réaction d'un acide carboxylique « néo » tel que décrit ci-dessus, avec un alcool qui peut être un mono-alcool ou un polyol.

Selon un mode de mise en oeuvre, l'alcool est un composé de formule R'OH telle que R'O est défini précédemment. Avantageusement, l'alcool comprend un nombre d'atomes de carbone allant de 2 à 20. L'alcool peut être notamment un alcool gras.

L'alcool peut être choisi parmi les mono-alcools comprenant de 16 à 20 atomes de carbone, linéaires ou ramifiés, saturés.

L'alcool peut également être un polyol choisi parmi les polyols « néo » c'est à dire les polyols qui contiennent un atome de carbone, situé en alpha d'une des fonctions alcool, qui est trisubstitué par des radicaux choisis indépendamment parmi les radicaux alkyle, aryle, aralkyle et leurs associations. Cet atome de carbone directement lié à 4 autres atomes de carbone est donc un carbone « néo » comme défini plus haut.
Comme polyol « néo », on choisit en particulier le néopentyl glycol de formule $HOCH_2$-$C(CH_3)_2$-$CH_2OH$, le triméthylol propane de formule $HOCH_2$-$C(C_2H_5)(CH_2OH)_2$, le pentaérythritol de formule $HOCH_2$-$C(CH_2OH)_2$-$CH_2OH$ et leurs mélanges. De façon préférentielle, le polyol est le néopentyl glycol.

L'ester hydrocarboné de la composition selon la présente invention peut représenter de 0,1 à 99,9 %, de préférence de 1 à 99% et mieux de 5 à 90% du poids total de la composition. Il représente par exemple de 5 à 60% du poids total de la composition.

HUILE

La phase grasse de la composition selon l'invention peut comprendre, outre l'ester hydrocarboné décrit précédemment au moins une huile. Par "huile", on entend tout milieu non aqueux liquide à température ambiante (25°C) et pression atmosphérique (760 mm de Hg), physiologiquement acceptable.

La quantité d'huile(s) peut aller par exemple de 20 à 80 % en poids et de préférence de 30 à 70 % en poids par rapport au poids total de la composition.

Ces huiles peuvent être des huiles hydrocarbonées et/ou siliconées et/ou fluorées. Elles peuvent être d'origine animale, végétale, minérale ou synthétique. On entend par « huile hydrocarbonée », toute huile comportant majoritairement des atomes de carbone et d'hydrogène, et éventuellement des groupements ester, éther, fluoré, acide carboxylique et/ou alcool. En outre, les huiles utilisées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25°C et 1 atmosphère, par exemple supérieure à 0 Pa, en particulier allant de $10^{-3}$ à 300 mm de Hg (0,13 Pa à 40.000 Pa). On peut notamment citer comme huiles volatiles, les huiles siliconées volatiles, telles que les silicones volatiles cycliques ou linéaires. On peut également citer les huiles volatiles hydrocarbonées telles que les isoparaffines, et les huiles fluorées volatiles.

Parmi les huiles pouvant être utilisées dans la composition de l'invention, certaines sont polaires et d'autres sont apolaires (c'est-à-dire non polaires).

Les huiles polaires comportent dans leur structure chimique au moins un groupement polaire non ionique, et de préférence au moins deux groupements polaires non ioniques ou ioniques tels que les groupements suivants :

- COOH;
- OH mono ou disubstitué (primaire ou secondaire) ;
- $PO_4$ ;
- NHR ; $NR_1R_2$, $R_1$ et $R_2$ formant éventuellement un cycle et représentant un radical alkyle ou alcoxy linéaire ou ramifié en $C_1$ à $C_{20}$, ou

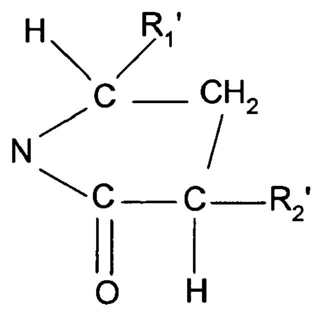

où $R_1$' et $R_2$' peuvent représenter l'hydrogène ou une chaîne alkyle ou alkoxy linéaire ou ramifiée en $C_1$ à $C_{20}$.

La polarité peut être décrite par le paramètre de solubilité de Hansen $\delta_a$. En effet, ce paramètre caractérise, pour un constituant donné, l'énergie correspondant aux interactions polaires ($\delta_p$) et de types liaisons hydrogène ($\delta_h$) existant entre les molécules de ce cons-

tituant.

$$\delta_a = \sqrt{\delta_p^2 + \delta_h^2}$$

Les huiles apolaires ont une valeur de $\delta_a$ égale à 0. En particulier, les huiles apolaires selon l'invention peuvent être en particulier choisies parmi :

- les huiles de silicone, telles que les polydiméthylsiloxanes (PDMS) volatils ou non, linéaires ou cycliques, liquides à température ambiante ; les silicones phénylées comme les phényltriméthicones, les phényldiméthicones, les phényltriméthylsiloxydiphénylsiloxanes, des diphényldiméthicones, les diphénylméthyldiphényltrisiloxanes, les 2-phényléthyl triméthylsiloxysilicates ;
- les hydrocarbures, linéaires ou ramifiés, d'origine synthétique ou minérale, comme les huiles de paraffine, volatiles ou non volatiles, et leurs dérivés ; l'huile de vaseline ; la lanoline liquide ; les polydécènes ; le polyisobutène hydrogéné tel que l'huile de Parléam® ; le squalane ; l'isoparaffine hydrogénée ; l'isohexadecane ; l'isododecane ;
- et leurs mélanges.

Les huiles polaires ont une valeur de $\delta_a$ différente de 0, c'est-à-dire supérieure à 0. En particulier, les huiles polaires utilisées dans la composition de l'invention peuvent être choisies parmi :

- les huiles d'origine végétale, huiles hydrocarbonées à forte teneur en triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées. Comme huiles d'origine végétale, on peut citer notamment les huiles de jojoba, de germes de blé, de maïs, de tournesol, de beurre de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, de rosier muscat, de coriandre ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse ou esters de synthèse de formule $R_5COOR_6$ dans laquelle $R_5$ représente le reste d'un acide gras linéaire ou ramifié comportant de 1 à 40 atomes de carbone et $R_6$ représente une chaîne hydrocarbonée notamment ramifiée contenant de 1 à 40 atomes de carbone à condition que $R_5 + R_6$ soit $\geq 10$, comme par exemple l'huile de Purcellin (octanoate de cétostéaryle), l'isononanoate d'isononyle, le benzoate d'alcool en $C_{12}$ à $C_{15}$, le myristate d'isopropyle, le palmitate d'éthyl 2-hexyle, l'isostéarate d'isostéaryle, l'isostéarate d'isopropyle, des octanoates, décanoates ou ricinoléates d'alcools ou de polyalcools ; les esters hydroxylés comme le lactate d'isostéaryle, le malate de di-isostéaryle ; le C12-C15 alkylbenzoate ; et les esters du pentaérythritol ;
- les éthers de synthèse ayant de 10 à 40 atomes de carbone ;
- les alcools gras en $C_8$ à $C_{26}$, comme l'alcool oléique, l'alcool isostéarylique, et l'octyldodécanol ;
- leurs mélanges.

PATEUX

La composition selon l'invention peut comprendre en outre au moins un composé pâteux différent de l'éther de polyol décrit précédemment.

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide. Par pâteux, on entend également le polylaurate de vinyle.

Le composé pâteux au sens de l'invention, peut avoir une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux peut être, à la température de 23°C, sous la forme d'une fraction liquide et d'une fraction solide. En d'autres termes, la température de fusion commençante du composé pâteux peut être inférieure à 23°C. La fraction liquide du composé pâteux mesurée à 23°C peut représenter 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état

liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi

- la lanoline et ses dérivés
- les composés siliconés polymères ou non
- les composés fluorés polymères ou non
- les polymères vinyliques, notamment:
  - les homopolymères d'oléfines
  - les copolymères d'oléfines
  - les homopolymères et copolymères de diènes hydrogénés
  - les oligomères linéaires ou ramifiés, homo ou copolymères de (méth)acrylates d'alkyles ayant de préférence un groupement alkyle en $C_8$-$C_{30}$
  - les oligomères homo et copolymères d'esters vinyliques ayant des groupements alkyles en $C_8$-$C_{30}$
  - les oligomères homo et copolymères de vinyléthers ayant des groupements alkyles en $C_8$-$C_{30}$,
- les polyéthers liposolubles résultant de la polyéthérification entre un ou plusieurs diols en C2-C100, de préférence en C2-C50,
- les esters,

et leurs mélanges.

Le composé pâteux est de préférence polymère, notamment hydrocarboné.

Un composé pâteux siliconé et fluoré préféré est le polyméthyl trifluoropropryl méthylalkyl diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Parmi les polyéthers liposolubles, on préfère en particulier les copolymères d'éthylène-oxyde et/ou de propylène-oxyde avec des alkylènes-oxydes à longue chaîne en C6-C30, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés
- les esters de pentaérythritol
- les polyesters non réticulés résultant de la polycondensation entre un acide dicarboxylique ou un polyacide carboxylique linéaire ou ramifié en C4-C50 et un diol ou un polyol en C2-C50,
- les esters aliphatiques d'ester résultant de l'estérification d'un ester d'acide hydroxycarboxylique aliphatique par un acide carboxylique aliphatique;

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

L'acide carboxylique aliphatique est de préférence ramifié.

L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :

a) les esters partiels ou totaux d'acides monocarboxyliques aliphatiques mono hydroxylés linéaires, saturés ;
b) les esters partiels ou totaux d'acides monocarboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en $C_2$ à $C_{16}$ ayant réagi avec un mono ou un poly acide carboxylique aliphatique mono ou poly hydroxylé,

et leurs mélanges.

Les esters aliphatiques d'ester sont avantageusement choisis parmi :

- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Le composé pâteux représente de préférence 1 à 99%, mieux 1 à 60%, mieux 2 à 30% et mieux encore 5 à 15% en poids de la composition.

CIRE

La phase grasse de la composition selon l'invention peut comprendre au moins une cire. Une cire, au sens de la présente invention, est un composé gras lipophile, solide à température ambiante (25°C), à changement d'état solide/liquide réversible, ayant une température de fusion supérieure à 40°C et pouvant aller jusqu'à 200°C, et présentant à l'état solide une organisation cristalline anisotrope.

On peut utiliser tout type de cire. Les cires peuvent être choisies parmi les cires d'origine naturelle, notamment d'origines végétale ou animale, parmi les cires d'origine minérale, parmi les cires d'origine synthétique, et leurs mélanges. Comme cires pouvant être utilisées dans la composition de l'invention, on peut citer par exemple la cire d'abeilles, la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège, les cires de paraffine, les cires microcristallines, la cire de lanoline, les ozokérites, les huiles hydrogénées ayant une température de fusion supérieure à 40°C (environ), comme l'huile de jojoba hydrogénée, les cires de polyéthylène qui sont issues de la polymérisation de l'éthylène, les cires obtenues par synthèse de Fischer-Tropsch, les esters d'acides gras et les glycérides concrets (c'est-à-dire solides) à 40°C, les cires de silicone comme les alkyle, alcoxy et/ou esters de poly(di)méthylsiloxane solides à 40°C ; et leurs mélanges.

Comme indiqué ci-dessus, quand la composition comprend une huile polaire, elle doit contenir au moins une cire apolaire. Quand la composition comprend une huile apolaire, elle doit contenir au moins une cire polaire. Quand la composition comprend une huile polaire et une huile apolaire, elle doit comprendre au moins une cire apolaire.

Comme indiqué ci-dessus pour les huiles, la polarité peut être décrite par le paramètre de solubilité de Hansen $\delta_a$ selon l'équation indiquée ci-dessus.

Les cires dites apolaires ont une valeur de $\delta_a$ égale à 0. Ce sont notamment les cires hydrocarbonées comportant essentiellement des atomes de carbone et d'hydrogène, ou des cires de silicone. Comme cires hydrocarbonées, on peut citer en particulier les cires microcristallines, l'ozokérite, les cires de paraffine, les cires de polyéthylène (non modifiées).

Les cires dites polaires sont des cires comportant des groupements polaires tels qu'indiqués ci-dessus pour les huiles, et elles ont une valeur de $\delta_a$ supérieure à 0. Ce sont notamment les cires d'origine animale, les cires d'origine végétale, les cires d'origine synthétique comportant des groupements polaires et les cires de silicone comportant des groupements polaires. On peut citer par exemple la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège, les cires d'abeille polyglycérolées, les huiles hydrogénées, les esters d'acides gras et les glycérides concrets à 40°C, les cires de silicone comportant un ou plusieurs groupes ester.

La quantité totale de cire(s) (polaires et/ou apolaires) peut aller par exemple de 5 à 40 % en poids et de

préférence de 10 à 30 % en poids par rapport au poids total de la composition.

La composition de l'invention peut comprendre de 0 à 10 % en poids d'une phase hydrophile, par rapport au poids total de la composition, et mieux de 1 à 5 % en poids, pouvant comprendre de l'eau et/ou des additifs hydrophiles ou hydrosolubles (actifs et/ou gélifiants par exemple). Elle peut notamment comprendre des hydratants tels que la glycérine. Les constituants hydrophiles éventuellement présents sont de préférence dispersés dans la phase grasse constituée des huiles et cires.

La composition selon l'invention peut comprendre en outre une phase particulaire. La quantité de phase particulaire peut aller par exemple de 0 à 30 % en poids, de préférence de 0 à 20 % en poids par rapport au poids total de la composition. Quand une phase particulaire est présente, sa quantité est généralement d'au moins 0,05 % en poids par rapport au poids total de la composition. La quantité de phase particulaire peut aller par exemple de 0,05 à 30 % en poids et de préférence de 1 à 20 % en poids par rapport au poids total de la composition. Cette phase particulaire peut comprendre des particules choisies parmi les pigments, les nacres, les charges, et leurs mélanges. Ces pigments, nacres, et charges sont choisis parmi ceux habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les pigments peuvent être blancs ou colorés, minéraux et/ou organiques, de taille micrométrique ou nanométrique. On peut citer par exemple, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Parmi les nacres utilisables dans la composition de l'invention, on peut citer par exemple le mica recouvert d'oxyde de titane, d'oxyde de fer, de pigment naturel ou d'oxychlorure de bismuth ainsi que le mica titane coloré.

Les charges peuvent être minérales ou de synthèse, lamellaires ou non lamellaires. Comme charges utilisables dans la composition de l'invention, on peut citer par exemple le talc, le mica, la silice, le kaolin, les poudres de Nylon, les poudres de polyéthylène, le Téflon, l'amidon modifié ou non, le micatitane, la nacre naturelle, le nitrure de bore, les microsphères telles que l'Expancel (Nobel Industrie), le Polytrap (Dow Corning) et les microbilles de résine de silicone (Tospearls de Toshiba, par exemple).

Avantageusement, la composition selon l'invention comprend au moins une matière colorante qui peut être choisie parmi les colorants lipophiles ou les colorants hydrophiles habituellement utilisés dans les compositions cosmétiques ou dermatologiques, ainsi que parmi les pigments et les nacres décrits ci-dessus ; et leurs mélanges. Cette matière colorante est généralement présente à raison de 0,01 à 40 % en poids et de préférence de 5 à 25 % en poids par rapport au poids total de la composition.

La composition selon l'invention peut comprendre en outre tout additif usuellement utilisé dans le domaine considéré, notamment le domaine cosmétique, tel que les antioxydants ; les parfums ; les huiles essentielles ; les conservateurs ; les actifs cosmétiques ; les vitamines comme la vitamine E (tocophérol) et ses dérivés (par exemple acétate), la vitamine A (rétinol) et ses dérivés (par exemple palmitate de rétinyle), la vitamine C (acide ascorbique) et ses dérivés (par exemple palmitate d'ascorbyle), les dérivés de ces vitamines étant notamment des esters dont le palmitate et l'acétate ; les acides gras essentiels ; les sphingolipides et céramides ; les composés auto-bronzants tels que la DHA (dihydroxyacétone) ; les filtres solaires comme par exemple l'octylméthoxycinnamate (Parsol MCX), la 3-benzophénone (Uvinul M40), le butylméthoxydibenzoylméthane (Parsol 1789) ; les tensioactifs ; les polymères ; et leurs mélanges. Ces additifs peuvent être présents dans la composition à raison de 0 à 20 % en poids par rapport au poids total de la composition.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les procédés de fabrication des compositions selon l'invention ne diffèrent en rien des procédés classiquement utilisés en cosmétique et parfaitement connus de l'homme de l'art.

Les compositions selon l'invention peuvent notamment constituer un produit de soin et/ou un produit de maquillage des matières kératiniques, notamment de la peau, des lèvres et des phanères comme les ongles, les cils, les sourcils et les cheveux. Les produits de maquillage sont le plus souvent colorés et contiennent généralement des pigments. Sous forme de produits de maquillage, les compositions de l'invention peuvent constituer avantageusement un fond de teint, un rouge à lèvres, un fard à joues, un fard à paupières, un mascara ou un eyeliner.

Ce produit peut être sous forme d'une poudre coulée, d'un produit en coupelle (fond de teint, fard à joues, fard à paupières), ou d'un produit sous forme de bâton (rouge à lèvres ou stick de soin des lèvres). Selon un mode préféré de réalisation de l'invention, elle se présente sous forme de bâton (stick), plus particulièrement pour le soin des lèvres ou pour le maquillage des lèvres

comme rouge à lèvres.

Aussi, l'invention a aussi pour objet l'utilisation cosmétique d'une composition cosmétique telle que définie ci-dessus, pour le soin et/ou le maquillage de la peau, des lèvres et/ou des phanères, et en particulier pour l'hydratation des lèvres.

L'invention a aussi pour objet l'utilisation cosmétique d'une composition cosmétique telle que définie ci-dessus, pour le soin et/ou le maquillage de la peau, des lèvres et/ou des phanères.

L'invention a aussi pour objet un procédé cosmétique de soin des lèvres gercées et/ou sèches, consistant à appliquer sur les lèvres, une composition cosmétique telle que définie ci-dessus.

L'invention est illustrée plus en détail dans les exemples suivants, dans lesquels les pourcentages sont donnés en poids, sauf mention contraire.

**<u>Exemple 1 : rouge à lèvres</u>**

| | |
|---|---|
| Lanolide ® (société Vevy) | 18,6 % |
| Néopentanoate d'isostéaryle | 13,3 |
| Bisglycéryl polyadipate (Softisan 100) | 3,35 |
| Phényl triméthicone | 7,6 |
| Phényl triméthicone 20 cSt | 3,6 |
| Bentone | 1,44 |
| Isostearate d'isopropyle | 22,56 |
| Ozokérite (cire minérale) | 1,75 |
| Cire de polyéthylene | 6,5 |
| Cire de polyéthylene | 3,2 |
| Dioxyde de titane | 0,73 |
| Red 7 | 3,4 |
| Yellow 6 lake | 6,12 |
| Kaolin | 5 |
| N-Lauroyl lysine | 2,5 |
| DL- bisabolol | 0,3 |
| BHT | qs |

Mode opératoire :

Broyage des pigments à la tricylindre avec la phase huileuse et la phase gel.
Le gel a été obtenu sous turbine jusqu'à gonflement et homogénéisation de la bentone. Les cires sont pesées dans un poêlon à double paroi à circulation d'huile, puis mises à chauffer sous agitation (turbine). Après fonte totale, le broyat, les phényltriméthicones et les actifs sont ajoutés. Après homogénéisation du mélange, le jus est coulé à 100°C dans un moule alu siliconé à 42°C. Après recristallisation des sticks à -4°C pendant au moins 30min, les raisins sont introduits dans les AC puis mis pour stabilisation pendant 24h à 20°C avant toute évaluation.

## Revendications

1. Composition cosmétique comprenant dans un milieu physiologiquement acceptable, au moins une phase grasse comportant au moins un éther de polyol, choisi parmi les éthers de pentaérythritol et de polyalkylène glycol, et au moins un ester résultant de la réaction d'un alcool aliphatique avec un acide carboxylique de formule (I) suivante :

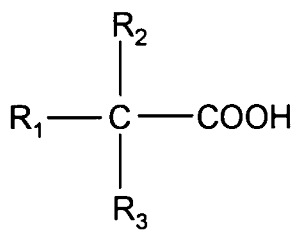

(I)

dans laquelle $R_1$, $R_2$ et $R_3$ sont des radicaux choisis indépendamment parmi les radicaux alkyle, aryle, aralkyle, et leurs associations.

2. Composition comprenant dans un milieu physiologiquement acceptable, au moins une phase grasse comportant au moins un éther de polyol, au moins un ester hydrocarboné et une huile non volatile différente dudit ester hydrocarboné.

3. Composition comprenant dans un milieu physiologiquement acceptable, au moins une phase grasse comportant au moins un éther de polyol et aux moins deux esters hydrocarbonés.

4. Composition comprenant dans un milieu physiologiquement acceptable, au moins une phase grasse comportant au moins un éther de polyol, un composé pâteux autre que ledit éther de polyol, et au moins un ester hydrocarboné.

5. Composition selon la revendication 1, **caractérisée en ce que** les radicaux $R_1$, $R_2$ et $R_3$ sont choisis indépendamment parmi les radicaux alkyle saturés en $C_1$-$C_6$.

6. Composition selon l'une des revendications 1 ou 5, **caractérisée en ce que** l'acide carboxylique comprend un nombre total d'atomes de carbone allant de 5 à 30.

7. Composition selon l'une des revendications 1, 5 ou 6, **caractérisée en ce que** l'acide carboxylique est un monoacide.

8. Composition selon la revendication précédente, **caractérisée en ce que** l'acide carboxylique est choisi parmi l'acide néopentanoïque, l'acide néoheptanoïque, l'acide néodécanoïque, et leurs mélanges.

**9.** Composition selon la revendication précédente, **caractérisée en ce que** l'acide carboxylique est l'acide néopentanoïque.

**10.** Composition selon la revendication 1 ou l'une des revendications 5 à 9, **caractérisée en ce que** l'alcool aliphatique est choisi parmi les mono-alcools ou les polyols.

**11.** Composition selon la revendication 1 ou l'une des revendications 5 à 10, **caractérisée en ce que** l'alcool comprend un nombres d'atomes de carbone allant de 2 à 20.

**12.** Composition selon la revendication 1 ou l'une des revendications 5 à 11, **caractérisée en ce que** l'alcool est choisi parmi les mono-alcools comprenant de 16 à 20 atomes de carbone, linéaires ou ramifiés, saturés.

**13.** Composition selon la revendication précédente, **caractérisée en ce que** l'ester est le néopentanoate d'isostéaryle.

**14.** Composition selon la revendication 1 ou l'une des revendications qui en dépendent, **caractérisée en ce qu'**elle contient outre l'ester d'alcool et d'acide de formule (I), un autre ester hydrocarboné.

**15.** Composition selon la revendication précédente, **caractérisée en ce que** l'ester hydrocarboné est un mono-ester ramifié et saturé.

**16.** Composition selon l'une des revendications 1 à 14, **caractérisée en ce que** l'ester de formule (I) et le mono-ester sont dans une proportion massique comprise entre 2/1 et 1/5, notamment comprise entre 1/1 et 1/5, de préférence entre 1/1 et 1/3, de préférence encore de l'ordre de 1/2.

**17.** Composition selon l'une des revendications 2, 3, 4, ou 14 à 16, **caractérisée en ce que** l'ester hydrocarboné comprend de 10 à 60, de préférence de 10 à 45, de préférence de 18 à 40 atomes de carbone.

**18.** Composition selon l'une des revendications 2, 3, 4, ou 14 à 17, **caractérisée en ce que** l'ester hydrocarboné est choisi parmi

- les esters de l'acide isononanoïque comme l'isononanoate d'isononyle, l'isononanoate d'octyle, l'isononanoate d'isodécyle, l'isononanoate d'isotridécyle, l'isononanoate d'isostéaryle,
- les esters de l'alcool isopropylique, tels que le myristate d'isopropyle, le palmitate d'isopropyle, le stéarate ou l'isostéarate d'isopropyle,
- les esters d'acides gras comme l'huile de purcellin, le palmitate d'éthyle, le stéarate d'octyle ;
- les esters hydroxylés comme le lactate d'isostéaryle, l'hydroxystéarate d'octyle, l'adipate de diisopropyle,
- les heptanoates, octanoates, décanoates d'alcool gras, comme l'octanoate de cétyle et l'octanoate de tridécyle
- et leurs mélanges.

**19.** Composition selon l'une des revendications précédentes, **caractérisée en ce que** l'éther de polyol est choisi parmi les éthers de pentaérythritol et de polyéthylène glycol comportant de 1 à 450 motifs oxyéthylénés, les éthers de pentaérythritol et de polypropylène glycol comportant de 1 à 450 motifs oxypropylénés, et leurs mélanges.

**20.** Composition selon la revendication précédente, **caractérisée en ce que** l'éther de polyol est choisi parmi l'éther de pentaérythritol et de polyéthylène glycol comportant 5 motifs oxyéthylénés, l'éther de pentaérythritol et de polypropylène glycol comportant 5 motifs oxypropylénés, et leurs mélanges.

**21.** Composition selon la revendication précédente, **caractérisée en ce que** l'éther de polyol est sous forme d'un mélange éther de pentaérythritol et de polyéthylène glycol comportant 5 motifs oxyéthylénés / éther de pentaérythritol et de polypropylène glycol comportant 5 motifs oxypropylénés / huile de soja.

**22.** Composition selon l'une quelconque des revendications précédentes, **caractérisée en ce que** l'éther de polyol est choisi parmi les éthers ou mélanges d'éthers d'alcool gras en $C_8$-$C_{22}$ et de glucose, de maltose, de sucrose ou de fructose ; les éthers ou mélanges d'éthers d'alcool gras en $C_{14}$-$C_{22}$ et de méthylglucose ; et leurs mélanges.

**23.** Composition selon l'une quelconque des revendications précédentes, **caractérisée en ce que** la quantité d'éther(s) de polyol va de 0,5 à 40 % en poids et de préférence de 1 à 30 % en poids par rapport au poids total de la composition.

**24.** Composition selon l'une des revendications précédentes se présentant sous forme de produit de maquillage du corps, d'un rouge ou brillant à lèvres, d'un mascara, d'un vernis à ongles, d'un produit de coloration ou de soin des cheveux, d'un déodorant.

**25.** Composition selon l'une des revendications précédentes, **caractérisée en ce qu'**elle se présente sous forme coulée ou compactée.

**26.** Composition selon l'une des revendications précédentes, **caractérisée en ce qu'**elle se présente sous forme d'un rouge à lèvres.

**27.** Composition selon l'une quelconque des revendications précédentes, **caractérisée en ce qu'**elle comprend une phase particulaire.

**28.** Composition selon la revendication précédente, **caractérisée en ce que** la phase particulaire comprend des particules choisies parmi les pigments, les nacres, les charges, et leurs mélanges.

**29.** Utilisation cosmétique d'une composition cosmétique selon l'une quelconque des revendications précédentes, pour le soin et/ou le maquillage de la peau, des lèvres et/ou des phanères, et en particulier pour l'hydratation de la peau, des lèvres et/ou des phanères.

**30.** Utilisation cosmétique d'une composition cosmétique selon l'une quelconque des revendications 1 à 28, pour le maquillage et/ou le soin de la peau, des lèvres et/ou des phanères.

**31.** Procédé cosmétique de soin des lèvres gercées et/ou sèches, consistant à appliquer sur les lèvres, une composition cosmétique selon l'une quelconque des revendications 1 à 28.

Office européen des brevets

# RAPPORT DE RECHERCHE EUROPEENNE

Numéro de la demande
EP 05 29 2051

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,X | US 2003/108579 A1 (YOUSFI NAIMA ET AL) 12 juin 2003 (2003-06-12)<br>* revendications 1,19; exemple 1 * | 1-31 | A61Q1/06<br>A61K8/86<br>A61K8/37 |
| A | DATABASE CA [Online] CHEMICAL ABSTRACTS SERVICE, COLUMBUS, OHIO, US; 2003, OMURA, TAKAYUKI ET AL: "Hair preparations containing polysiloxane-containing amphoteric polyurethanes" XP002330620 extrait de STN Database accession no. 2003:460520<br>* abrégé *<br>& JP 2003 171236 A2 (SHISEIDO CO., LTD., JAPAN) 17 juin 2003 (2003-06-17) | 1-31 | |
| A | FR 2 838 049 A (L'OREAL) 10 octobre 2003 (2003-10-10)<br>* revendications 1-40 * | 1-31 | **DOMAINES TECHNIQUES RECHERCHES (IPC)** |
| A | US 5 560 917 A (COHEN ET AL) 1 octobre 1996 (1996-10-01)<br>* revendication 1; tableau 2 *<br>& JP 2003 171236 A2 (SHISEIDO CO., LTD., JAPAN) 17 juin 2003 (2003-06-17) | 1-31 | A61K |
| A | US 6 372 201 B1 (LEURIDAN FR&EACUTE ET AL) 16 avril 2002 (2002-04-16)<br>* revendications 1,14,15,24; exemples 1,2 * | 1-31 | |
| A | EP 1 216 685 A (JOHNSON & JOHNSON CONSUMER COMPANIES, INC) 26 juin 2002 (2002-06-26)<br>* revendications 1,25,36,48 * | 1-31 | |
| P,A | WO 2005/018584 A (L'OREAL; ARNAUD, PASCAL) 3 mars 2005 (2005-03-03)<br>* revendications 1,18,20,22,41 * | 1-31 | |

Le présent rapport a été établi pour toutes les revendications

3

| Lieu de la recherche | Date d'achèvement de la recherche | Examinateur |
|---|---|---|
| La Haye | 28 février 2006 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 2051

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-02-2006

| Document brevet cité au rapport de recherche | Date de publication | Membre(s) de la famille de brevet(s) | Date de publication |
|---|---|---|---|
| US 2003108579 A1 | 12-06-2003 | CA 2406365 A1 | 25-04-2003 |
| | | CN 1413568 A | 30-04-2003 |
| | | EP 1306074 A1 | 02-05-2003 |
| | | FR 2831433 A1 | 02-05-2003 |
| | | JP 2003137724 A | 14-05-2003 |
| JP 2003171236 A2 | -- | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 2051

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-02-2006

| Document brevet cité au rapport de recherche | Date de publication | Membre(s) de la famille de brevet(s) | Date de publication |
|---|---|---|---|
| JP 2003171236 A2 | | --<br>--<br>--<br>--<br>--<br>--<br>--<br>--<br>--<br>-- | |
| FR 2838049 A | 10-10-2003 | AUCUN | |
| US 5560917 A | 01-10-1996 | AU 4972796 A<br>EP 0806937 A1<br>JP 10513188 T<br>WO 9623484 A1 | 21-08-1996<br>19-11-1997<br>15-12-1998<br>08-08-1996 |
| JP 2003171236 A2 | -- | --<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>-- | |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 2051

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-02-2006

| Document brevet cité au rapport de recherche | Date de publication | Membre(s) de la famille de brevet(s) | Date de publication |
|---|---|---|---|
| JP 2003171236 A2 | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| US 6372201 B1 | 16-04-2002 | AT 215350 T | 15-04-2002 |
| | | BR 0001105 A | 24-04-2001 |
| | | CA 2302854 A1 | 01-10-2000 |
| | | CN 1279058 A | 10-01-2001 |
| | | DE 60000103 D1 | 08-05-2002 |
| | | DE 60000103 T2 | 14-08-2002 |
| | | EP 1040814 A1 | 04-10-2000 |
| | | ES 2174801 T3 | 16-11-2002 |
| | | FR 2791561 A1 | 06-10-2000 |
| | | JP 2000290141 A | 17-10-2000 |
| EP 1216685 A | 26-06-2002 | AU 9735901 A | 27-06-2002 |
| | | CA 2365818 A1 | 21-06-2002 |
| | | CN 1366874 A | 04-09-2002 |
| | | JP 2002322045 A | 08-11-2002 |
| WO 2005018584 A | 03-03-2005 | AU 2003273476 A1 | 10-03-2005 |

EPO FORM P0460

Pour tout renseignement concernant cette annexe : voir Journal Officiel de l'Office européen des brevets, No.12/82